(19) Europäisches Patentamt
European Patent Office
Office européen des brevets

(11) EP 0 608 753 B1

(12) # EUROPÄISCHE PATENTSCHRIFT

(45) Veröffentlichungstag und Bekanntmachung des Hinweises auf die Patenterteilung:
**22.04.1998 Patentblatt 1998/17**

(21) Anmeldenummer: **94100643.9**

(22) Anmeldetag: **18.01.1994**

(51) Int Cl.[6]: **C07D 249/18**, C07D 491/04 // A01N43/82,(C07D491/04, 317:00, 249:00), (C07D491/04, 319:00, 249:00)

(54) **Fluorierte Benzotriazole verwendbar als Schädlingsbekämpfungsmittel**

Fluorinated benzotriazoles useful as pesticides

Benzotriazoles fluorés utiles comme pesticides

(84) Benannte Vertragsstaaten:
**CH DE FR GB LI NL**

(30) Priorität: **29.01.1993 DE 4302461**

(43) Veröffentlichungstag der Anmeldung:
**03.08.1994 Patentblatt 1994/31**

(73) Patentinhaber: **BAYER AG**
**51368 Leverkusen (DE)**

(72) Erfinder:
- **Marhold, Albrecht, Dr.**
  **51373 Leverkusen (DE)**
- **Baasner, Bernd, Dr.**
  **51467 Bergisch Gladbach (DE)**
- **Hänssler, Gerd, Dr.**
  **51381 Leverkusen (DE)**

(56) Entgegenhaltungen:
**EP-A- 0 108 908** **EP-A- 0 178 708**
**EP-A- 0 355 049** **EP-A- 0 367 242**

- **CHEMICAL ABSTRACTS, vol. 60, no. 2, 20. Januar 1964, Columbus, Ohio, US; abstract no. 1732d, L.M. YAGUPOL'SKII ET AL. 'Synthesis of 5-methyl-6-trifluoromethylbenzimidazole'**
- **CHEMICAL ABSTRACTS, vol. 73, no. 17, 26. Oktober 1970, Columbus, Ohio, US; abstract no. 87890u, G.A. VAVILOV ET AL. 'Ribiflavine analogs. VI. 7-Trifluoromethylisoalloxazines' Seite 358 ;**
- **CHEMICAL ABSTRACTS, vol. 119, no. 19, 8. November 1993, Columbus, Ohio, US; abstract no. 203733q, B.G. HUANG ET AL 'Synthesis of acyclic analogs of benzotriazole and indole nucleoside containing perfluoroalkyl group' Seite 934 ;**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 608 753 B1

Printed by Jouve, 75001 PARIS (FR)

## Beschreibung

Gegenstand der Erfindung sind Benzotriazole, die pro Molekül mindestens 2 Fluoratome enthalten und der Formel

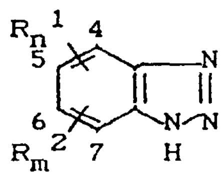

(I)

entsprechen, worin

| | |
|---|---|
| n, m = | 1 oder 2, |
| $R^1$ | für Fluor-$C_1$-$C_6$-alkoxy, Fluor-$C_1$-$C_6$-alkyllthio, und |
| $R^2$ | für Wasserstoff, Fluor, Chlor, Brom, Nitril, $C_1$-$C_6$-Alkyl, $C_1$-$C_6$-Alkoxy, $C_1$-$C_6$-Alkylthio, $C_1$-$C_6$-Alkylsulfonyl, $C_1$-$C_4$-Fluoralkyl, Fluor-$C_1$-$C_6$-alkoxy, Fluor-$C_1$-$C_6$-alkylthio, Fluor-$C_1$-$C_6$-alkylsulfonyl oder |
| $R^1$ und $R^2$ | gemeinsam für -O-X-O- stehen, worin X einen fluorierten $C_1$-$C_2$-Aklylenrest bedeutet, |

Die Substituenten $R^1$ und $R^2$ stehen - falls n = m = 1 - vorzugsweise in 4,6-, 5,6- und 5,7-Position.

Die Begriffe "Fluoralkyl", "Fluoralkoxy", "Fluoralkylthio" schließen eine zusätzliche Substitution dieser Reste durch Chlor nicht aus.

Der Begriff "Fluor-$C_1$-$C_6$-alkoxy" umfaßt vorzugsweise Difluormethoxy, Trifluormethoxy, Chlordifluormethoxy, 1,1,2,2-Tetrafluorethoxy, 2-Chlor-1,1,2-trifluorethoxy, Pentafluorethoxy und 1,1,2,3,3,3-Hexafluorpropoxy.

Der Begriff "Fluor-$C_1$-$C_6$-alkylthio" umfaßt vorzugsweise Difluormethylthio, Trifluormethylthio, Chlordifluormethylthio, 1,1,2,2-Tetrafluorethylthio, 2-Chlor-1,1,2-trifluorethylthio, Pentafluorethylthio und 1,1,2,3,3,3-Hexafluorpropylthio.

Der Begriff "Fluor-$C_1$-$C_6$-alkylsulfonyl" umfaßt vorzugsweise Difluormethylsulfonyl, Trifluormethylsulfonyl, Chlordifluormethylsulfonyl, 1,1,2,2-Tetrafluorethylsulfonyl, 2-Chlor-1,1,2-trifluorethylsulfonyl, Pentafluorethylsulfonyl und 1,1,2,3,3,3-Hexafluorpropylsulfonyl.

Bevorzugte Verbindungen I entsprechen den Formeln

(IV) 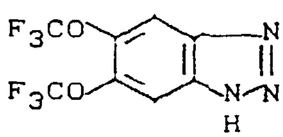

(V) 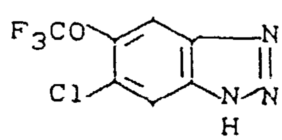

(VI) 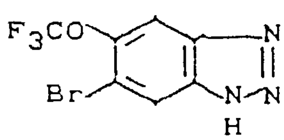

(VII) 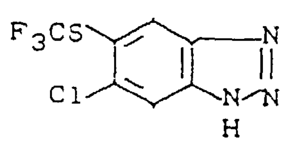

(VIII) 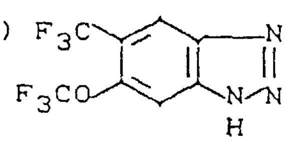

(X) 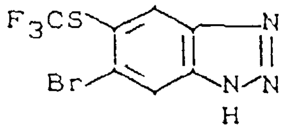

(XI) 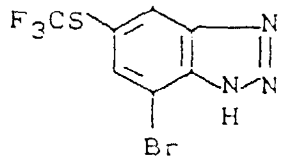

(XII) 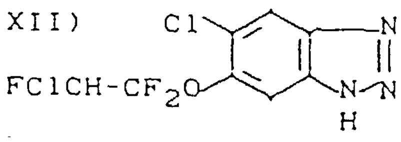

(XIII) 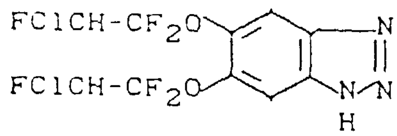

(XIV) 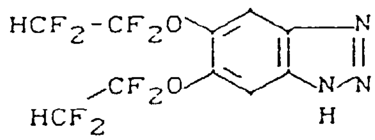

(XV) 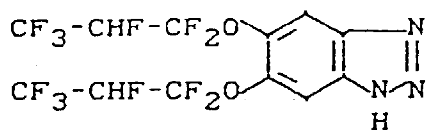

(XVI) 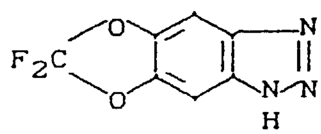

(XVII) 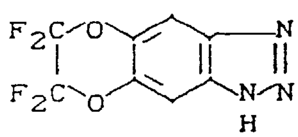

(XVIII) 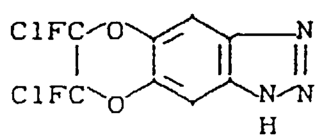

(XIX) 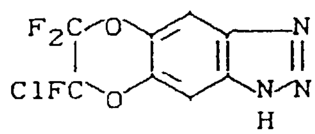

Die erfindungsgemäßen fluorierten Benzotriazole können durch Reaktion der entsprechenden o-Phenylendiamine mit einem Nitrosierungsmittel (z.B. Natriumnitrit) erhalten werden.

Die Kondensation der o-Phenylendiamine mit einem Nitrosierungsmittel erfolgt vorzugsweise in Lösung. Als Lösungsmittel oder Dispergier- bzw. Verdünnungsmittel kommen prinzipiell alle Lösemittel in Frage, die unter den Reaktionsbedingungen inert sind. Es können auch Mischungen von Lösemittel verwendet werden. Beispielhaft seien genannt: Wasser, Alkohole, Ketone, organische Säuren wie Ameisensäure, Essig- und Propionsäure, DMF, Toluol, Chlorbenzol, Dichlormethan, Trichlorethylen und ähnliche.

Vorteilhaft ist z.B. Wasser in Verbindung mit einer organischen Säure, da die Reaktion im sauren pH-Bereich durchgeführt wird.

Die Wahl des Löse-/Verdünnungsmittels richtet sich aber auch nach der Art des Nitrosierungsmittel. Wird beispielsweise ein Alkalinitrit wie Natriumnitrit verwendet, so ist dieses in Wasser besonders gut löslich und läßt diese Durchführung vorteilhaft erscheinen. Ebenso kann mit anderen Nitrosierungsmitteln wie Methyl- oder iso-Amylnitrit auch in anderen Lösungsmitteln gearbeitet werden.

Weitere Nitrosierungsmittel sind Stickoxide, Nitrosylschwefelsäure oder Nitrosylchlorid.

Die Zugabe des Nitrosierungsmittels erfolgt bevorzugt bei niedriger Temperatur, das heißt bei 0 bis 10°C, kann aber auch bei Temperaturen darunter oder darüber ausgeführt werden. Ebenso ist es möglich, die Reaktion bei erhöhter Temperatur zu Ende zu führen. Hierfür erwies sich ein Temperaturbereich von 40 bis 100°C als vorteilhaft.

Um zu einem möglichst reinen Produkt zu kommen, wird das Nitrosierungsmittel im molaren Mengenverhältnis oder in einem leichten Überschuß eingesetzt. Auf diese Weise wird ein vollständiger Umsatz erreicht und es entfällt die Trennung vom Ausgangsmaterial und Produkt. Ein größerer Überschuß an Nitrosierungsmittel stört nicht und kann aufgrund der sehr unterschiedlichen Löslichkeit leicht abgetrennt werden.

Für die Aufarbeitung des Ansatzes ist es günstig, ein Reaktionsmedium zu wählen, in dem das Produkt schlecht löslich ist, so daß es beispielsweise durch Filtration abgetrennt werden kann.

Die als Ausgangsprodukte geeigneten o-Phenylendiamine können, unabhängig von der Art der Reste $R^1$ und $R^2$, auf verschiedene Weise hergestellt werden:

Sollen o-Phenylendiamine hergestellt werden, bei denen sowohl $R^1$ als auch $R^2$ eine Donorgruppe in 4-und 5-Stellung darstellen, z.B. Verbindungen, bei denen $R^1$ für Polyfluoralkoxy oder Polyfluoralkylthio und $R^2$ für Fluor, Chlor, Brom, Alkyl oder Alkoxy stehen, so kann man ein Benzolderivat der Formel

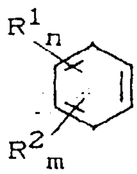

(XXIII),

dinitrieren und die Nitrogruppen anschließend reduzieren. Die Dinitrierung kann z.B. mit $HNO_3/H_2SO_4$-Gemischen, die gegebenenfalls auch Oleum enthalten können, bei Temperaturen von z.B. 0 bis 100°C durchgeführt werden. Die Reduktion kann beispielsweise mit Eisen in Gegenwart von wäßriger Salzsäure und Ethanol bei Temperaturen von z. B. 50 bis 100°C oder katalytisch mit elementarem Wasserstoff, z.B. 1 bis 100 bar und in Gegenwart von Metall oder Metallverbindungen der 8. Nebengruppe des periodischen Systems, insbesondere Nickel oder Palladium, enthaltenden Katalysatoren bei z.B. 25 bis 100°C durchgeführt werden.

Sollen o-Phenylendiamine hergestellt werden, bei denen $R^1$ die zu Formel (I) angegebene Bedeutung hat und in 4-Stellung steht und $R^2$ für Cl oder Br in 5-Stellung steht, so kann man z.B. ein Nitrobenzolderivat der Formel

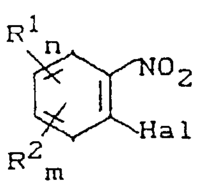

(XXIV),

in der

Hal für Fluor, Chlor oder Brom steht,

mit Ammoniak umsetzen, so die Hal-Gruppe gegen eine Aminogruppe austauschen und das so erhaltene Nitranilin reduzieren. Der Austausch von Halogen gegen eine Aminogruppe kann z.B. mit flüssigem Ammoniak in Gegenwart von Wasser und einem Tetraalkylammoniumsalz bei Temperaturen von z.B. 80 bis 200°C in einem Druckgefäß durchgeführt werden. Die Reduktion des Nitranilins kann z.B. analog zu der oben beschriebenen Reduktion von Dinitroverbindungen erfolgen.

Sollen o-Phenylendiamine hergestellt werden, bei denen $R^1$ die zu Formel (I) angegebene Bedeutung hat und in 4-Stellung steht und $R^2$ für Chlor oder Brom in 6-Stellung steht, so kann man z.B. ein Nitranilin der Formel

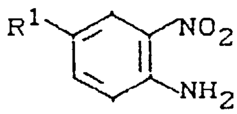

(XXV),

mit einem Chlorierungs- oder Bromierungsmittel umsetzen, so ein Chlor- oder Bromatom in die meta-Stellung zur Nitrogruppe einführen und anschließend die Nitrogruppe reduzieren. Als Chlorierungs- bzw. Bromierungsmittel kommen elementares Chlor, elementares Brom und andere übliche Chlorierungs- und Bromierungsmittel in Frage. Geeignete Lösungsmittel sind beispielsweise Wasser, verdünnte Mineralsäuren, Essigsäure, Chloralkane und Trifluoressigsäure und geeignete Temperaturen beispielsweise solche von -20 bis +50°C. Die Reduktion kann z.B. analog zu der oben beschriebenen Reduktion von Dinitroverbindungen erfolgen.

Drei- und vierfach substituierte o-Phenylendiamine können analog den oben geschilderten Verfahren hergestellt werden.

Die meisten der oben angesprochenen o-Phenylendiamine und Verfahren zu ihrer Herstellung werden in der deutschen Patentanmeldung P 42 37 564.9 und in der EP-A 251 013 beschrieben.

Die erfindungsgemäßen fluorierten Benzotriazole sind wertvolle Zwischenprodukte für die Herstellung von Wirkstoffen für Pharmazeutika und Pflanzenschutzmittel.

Die erfindungsgemäßen Verbindungen der Formel (I) weisen auch selbst eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide, geeignet.

Aus der EP-A 108 908 sind substituierte Benzotriazole und ihre Verwendung als biozide Wirkstoffe bekannt, wobei als Fluor-haltige Substituenten neben dem Fluoratom selbst lediglich noch Trifluormethyl genannt wird.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft, aber nicht begrenzend, seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubenis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea;
(Konidienform: Drechslera, Syn. Helminthosporium);
Cochliobolus-Arten, wie beispielsweise Cochliobolus sativus;
(Konidienform: Drechslera, Syn. Helminthosphorium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweie Leptosphaeria nodorum;
Cercospora-Arten, wie beispielsweise Cercospora canescens;
Alternaria-Arten, wie beispielsweise Alternaria brassicae;

Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei lassen sich die erfindungsgemäßen Wirkstoffe mit besonders gutem Erfolg zur Bekämpfung von Phytophthora an Tomaten sowie von Venturia-Arten an Äpfeln einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit und Diatomeenerde und synthetische Gesteinsmehle, wie hochdisperse Kiselsäure, Aluminiumoxid und Silikate; als feste Trägerstoffe für Granulate kommen in Frage; z.B. gebrochene und franktionierte natürliche Gesteine wie Calcit, Mamor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel: als Emulgier- und/oder schaumerzeugende Mittel kommen in Frage: z.B. nichtionogene und anionische Emulgatoren, wie Polyethylen-Fettalkohole-Ether, z.B. Alkyl-arylpolyglykol-ether, Alkylsufonate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummi arabicum, Polyvinylalkohol, Polyvinylacetat, sowie natürliche Phospholipide, wie Kephaline und Lecithine, und synthetische Phospholipide. Weitere Additive können mineralische und vegatabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen 0,0000001 bis 95 Gew.-% Wirkstoff, vorzugsweise 0,0001 bis 90 Gew.-%.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,0001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Beispiele

Herstellung von Ausgangsmaterialien (o-Phenylendiamine) für die neuen Benzotriazole

a) Zu 500 g einer Mischsäure enthaltend 33 Gew.-% $HNO_3$ und 67 Gew.-% $H_2SO_4$ wurden 320 g 1,2-Bis-(2-chlor-1,1,2-trifluorethoxy)-benzol getropft. Nach einer Stunde bei 40°C wurden 250 ml 20 gew.-%iges Oleum zugetropft. Anschließend wurde auf 80°C erhitzt und 15 Stunden lang nachgerührt. Dann wurden weitere 120 ml 20 gew.-%iges Oleum und 250 g der oben angegebenen Mischsäure zugetropft. Nach 6 Stunden bei 80 bis 82°C wurde abgekühlt und auf Eis gegossen. Die organische Phase wurde abgetrennt und mit Wasser gewaschen. Nach azeotroper Trocknung mit 1,2-Dichlorethan wurden 350 g 98 Gew.-% reines 1,2-Dinitro-4,5-bis-(2-chlor-1,1,2-trifluorethoxy)benzol erhalten (Öl, $n_D^{20}$ : 1,4832, GC 99,1%).

350 g dieser Dinitroverbindung wurden zu einem Gemisch aus 1,5 l Ethanol, 50 ml Wasser, 30 ml konzentrierter wäßriger Salzsäure und 470 g Eisenspänen getropft und insgesamt 15 Stunden zum Sieden am Rückfluß erhitzt. Danach wurde die erkaltete Lösung abfiltriert, eingeengt und der Rückstand aus Cyclohexan umkristallisiert. Es wurden 216 g 1,2 Diamino-4,5-bis-(2-chlor-1,1,2-trifluorethoxy)-benzol mit einem Schmelzpunkt von 58 bis 60°C erhalten.

b) 24 g fein gepulvertes 2-Nitro-4-trifluormethylmercapto-anilin wurden in 50 ml Trifluoressigsäure gelöst und bei 20°C 18 g Brom zudosiert. Dann wurde für 3 Stunden bei 20°C und für weitere 30 Minuten bei 40°C nachgerührt, die Mischung auf Wasser gegeben und das Produkt in Dichlormethan aufgenommen. Es fielen nach Entfernung des Lösungsmittels 31 g 6-Brom-2-nitro-4-trifluormethylmercapto-anilin an.

155 g des so hergestellten Nitranilins wurden in 700 ml Ethanol mit 15 ml Wasser, 10 ml konzentrierter wäßriger Salzsäure und 70 g Eisenspänen für 15 Stunden zum Sieden am Rückfluß erhitzt, dann das Gemisch abfiltriert, das Filtrat unter reduziertem Druck vom Lösungsmittel befreit und das feste Rohprodukt aus Cyclohexan umkristallisiert. Es wurden 112 g 6-Brom-4-trifluormethylmercapto-1,2-diaminobenzol mit einem Schmelzpunkt von 60 bis 61°C erhalten.

Analog können die übrigen o-Phenylendiamine hergestellt werden, die für die Herstellung der erfindungsgemäßen Benzotriazole benötigt werden.

Benzotriazol XVI

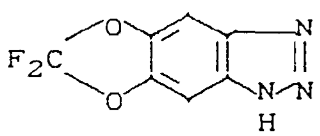

(XVI)

Zu einer Mischung aus 75 ml Wasser und 6 ml Eisessig wurden 10 g Diamin der Formel

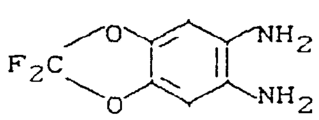

gegeben und 30 Minuten gerührt. Nach Abkühlen auf 0°C wurden 8 g Natriumnitrit, gelöst in 25 ml Wasser, rasch zugetropft. Anschließend wurde für 1 Stunde bei 0 bis 5°C und für 3 Stunden bei 80°C gerührt. Nach Abkühlen auf Raumtemperatur wurde der Feststoff abgesaugt und mit etwas Wasser nachgewaschen. Nach Trocknen fielen 9,6 g des gewünschten Produkts mit Schmelzpunkt 203 bis 204°C an.

Analog wurden erhalten:

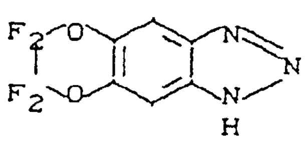

F: 147-148° C

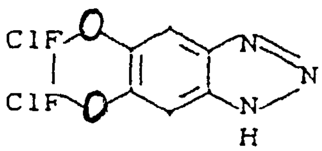

F: 151-152° C

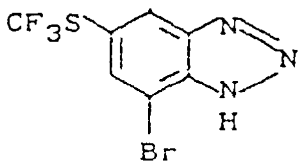

F: 228-229° C

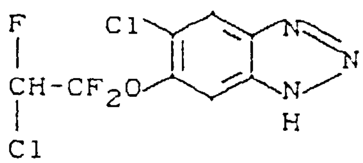

F: 159-160° C

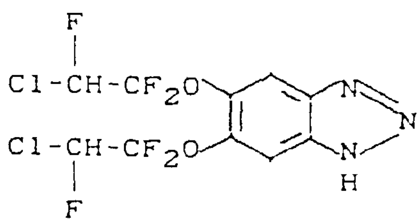

F: 153-154° C

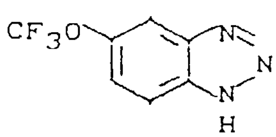

F: 107-109° C

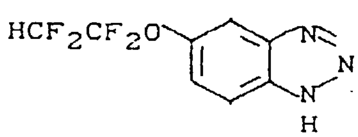

F: 77-78° C

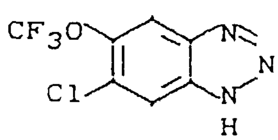

F: 124-125° C

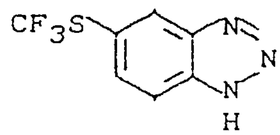

F: 125-127° C

Anwendung

Venturia-Test (Apfel) / protektiv

Lösungsmittel: 12,5 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in eienr Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele:

Tabelle

Venturia - Test (Apfel) / protektiv

| Wirkstoff | Wirkungsgrad in % der unbehandelten Kontrolle bei einer Wirkstoffkonzentration von 0,025 Gew.-% |
|---|---|
| 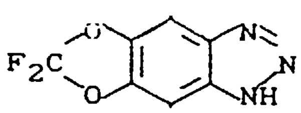 | 100 |
| 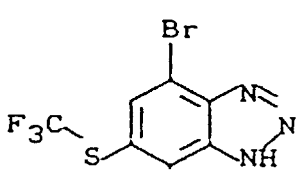 | 78 |
| 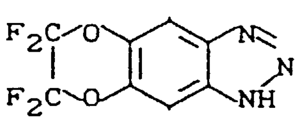 | 89 |

**Patentansprüche**

1. Benzotriazole, die pro Molekül mindestens 2 Fluoratome enthalten und der Formel

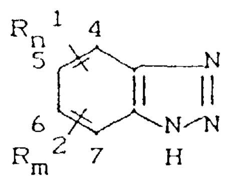

(I)

entsprechen, worin

n, m = 1 oder 2,

$R^1$ für Fluor-$C_1$-$C_6$-alkoxy, Fluor-$C_1$-$C_6$-alkyllthio und

$R^2$ für Wasserstoff, Fluor, Chlor, Brom, Nitril, $C_1$-$C_6$-Alkyl, $C_1$-$C_6$-Alkoxy, $C_1$-$C_6$-Alkylthio, $C_1$-$C_6$-Alkylsulfonyl, $C_1$-$C_4$-Fluoralkyl, Fluor-$C_1$-$C_6$-alkoxy, Fluor-$C_1$-$C_6$-alkylthio, Fluor-$C_1$-$C_6$-alkylsulfonyl oder

$R^1$ und $R^2$ gemeinsam für -O-X-O- stehen, worin X einen fluorierten $C_1$-$C_2$-Aklylenrest bedeutet.

2. Verfahren zur Herstellung der Benzotriazole nach Anspruch 1, wonach man die entsprechenden o-Phenylendiamine

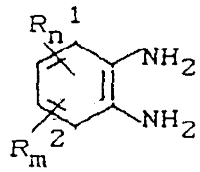

in saurer wäßriger Lösung mit einem Nitrosierungsmittel umsetzt.

3. Verwendung von Benzotriazolen nach Anspruch 1 als Zwischenprodukte für die Herstellung von Wirkstoffen für Pharmazeutika und Pflanzenschutzmittel.

4. Verwendung von Benzotriazolen nach Anspruch 1 als Wirkstoffe in Pflanzenschutzmitteln.

**Claims**

1. Benzotriazoles which contain at least 2 fluorine atoms per molecule and correspond to the formula

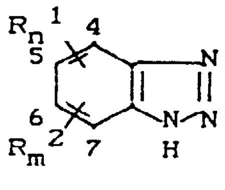

(I)

in which

n and m = 1 or 2,

$R^1$ represents fluoro-$C_1$-$C_6$-alkoxy, fluoro-$C_1$-$C_6$-alkylthio, and

$R^2$ represents hydrogen, fluorine, chlorine, bromine, nitrile, $C_1$-$C_6$-alkyl, $C_1$-$C_6$-alkoxy, $C_1$-$C_6$-alkylthio, $C_1$-$C_6$-alkylsulphonyl, $C_1$-$C_4$-fluoroalkyl, fluoro-$C_1$-$C_6$-alkoxy, fluoro-$C_1$-$C_6$-alkylthio or fluoro-$C_1$-$C_6$-alkylsulphonyl, or

$R^1$ and $R^2$ together represent -O-X-O-, in which X denotes a fluorinated $C_1$-$C_2$-alkylene radical,

**2.** Process for preparing the benzotriazoles according to Claim 1, according to which the corresponding o-phenylenediamines

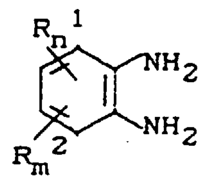

are reacted in acidic aqueous solution with a nitrosating agent.

**3.** Use of benzotriazoles according to Claim 1 as intermediates for the production of active compounds for pharmaceuticals and plant production agents.

**4.** Use of benzotriazoles according to Claim 1 as active compounds in plant protection agents.

**Revendications**

**1.** Benzotriazoles contenant au moins deux atomes de fluor par molécule et répondant à la formule

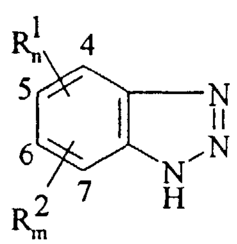

(I)

dans laquelle

n, m = 1 ou 2
$R^1$ représente un groupe fluoro-alcoxy en $C_1$-$C_6$, fluoro-alkylthio en $C_1$-$C_6$ et
$R^2$ représente l'hydrogène, le fluor, le chlore, le brome, un groupe nitrile, alkyle en $C_1$-$C_6$, alcoxy en $C_1$-$C_6$, alkylthio en $C_1$-$C_6$, alkylsulfonyle en $C_1$-$C_6$, fluoralkyle en $C_1$-$C_4$, fluoro-alcoxy en $C_1$-$C_6$, fluoro-alkylthio en $C_1$-$C_6$, fluoroalkylsulfonyle en $C_1$-$C_6$,
ou bien
$R^1$ et $R^2$ forment ensemble -O-X-O-, dans lequel X représente un groupe alkylène en $C_1$-$C_2$ fluoré.

**2.** Procédé de préparation des benzotriazoles de la revendication 1, selon lequel on fait réagir les o-phénylènediamines correspondantes

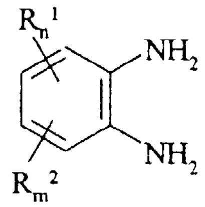

en solution aqueuse acide, avec un agent nitrosant.

**3.** Utilisation des benzotriazoles selon la revendication 1 en tant que produits intermédiaires de la préparation de substances actives pour produits pharmaceutiques et phytosanitaires.

**4.** Utilisation des benzotriazoles selon la revendication 1 en tant que substances actives de produits phytosanitaires.